# EUROPÄISCHE PATENTSCHRIFT

(11) **EP 1 001 711 B1**
(45) Veröffentlichungstag und Bekanntmachung des Hinweises auf die Patenterteilung: **29.12.2004**
(21) Anmeldenummer: 98945202.4
(22) Anmeldetag: 10.08.1998
(51) Int. Cl.: A61B 18/00

(54) **VORRICHTUNG FÜR DIE HOCHFREQUENZBEHANDLUNG VON KÖRPERGEWEBE**
DEVICE FOR THE HIGH-FREQUENCY TREATMENT OF BODY TISSUE
DISPOSITIF POUR LE TRAITEMENT HAUTE FREQUENCE DE TISSUS CORPORELS

(30) Priorität: 08.08.1997 DE 19734506
(43) Veröffentlichungstag der Anmeldung: 24.05.2000
(73) Patentinhaber: Stockert GmbH, 79104 Freiburg (DE)
(72) Erfinder: STOCKERT, Rüdiger, D-79104 Freiburg (DE)
(74) Vertreter: von Hellfeld, Axel, Dr. Dipl.-Phys.
(86) Internationale Anmeldenummer: PCT/EP1998/005068
(87) Internationale Veröffentlichungsnummer: WO 1999/007298

(56) Entgegenhaltungen:
- WO-A-92/02272
- NL-C- 1 004 655

## Beschreibung

Die Erfindung betrifft eine Vorrichtung für die Hochfrequenzbehandlung von Körpergewebe mit einem Kopf und wenigstens einer HF-Elektrode aus elektrisch leitfähigem Material, die mit dem zu behandelnden Gewebe in Kontakt bringbar ist.

Eine Vorrichtung für die Hochfrequenzbehandlung, insbesondere Koagulation, ist aus der DE 3930451 A1 bekannt. Der dort gezeigte Kopf der Vorrichtung hat eine vordere Elektrode am distalen Ende und in proximaler Richtung dazu beabstandet eine hintere Elektrode. Zwischen den Elektroden ist ein elektrisch isolierender Ring aus Kunststoff angeordnet.

Auch die DE 3511107 C2 beschreibt eine Vorrichtung für die Hochfrequenzkoagulation von biologischem Gewebe. Dort sind die Schenkel einer Koagulationspinzette aus elektrisch leitendem Metall gebildet.

Bei der in der DE 3838840 C2 beschriebenen Vorrichtung für die Hochfrequenzbehandlung von Körpergewebe (HF-Koagulationsvorrichtung) ist der Kopf aus einem Mantel aus schlecht wärmeleitendem Stahl gebildet, auf den eine Al/Ag-Schicht als Elektrode aufgebracht ist.

Die DE 3510586 C2 beschreibt ebenfalls ein Hochfrequenz-Chirurgiegerät. Dort ist eine monopolare aktive Elektrode vorgesehen, die mit einer neutralen Elektrode zusammenwirkt. Alternativ ist auch eine bipolare Elektrode vorgesehen.

Zum Stand der Technik wird noch auf folgende Schriften verwiesen: EP 0 246 350 A1, GB 2308979 A, DE 30 50 386 C2, und WO 95/10978 A1.

Eine Vorrichtung gemäß der Präambel des Anspruchs 1 ist aus dem Dokument WO-A-92/02272 bekannt.

Die vorliegende Erfindung kann sowohl bei monopolaren Elektroden als auch bei bipolaren Elektroden eingesetzt werden.

Der vorstehend genannte Stand der Technik wird nachfolgend als bekannt vorausgesetzt, insbesondere die elektrische Versorgung und die Steuerung der HF-Energie.

Die vorliegende Erfindung betrifft eine besondere Gestaltung des Kopfes eines HF-Chirurgiegerätes. Unter dem Kopf ist dasjenige Bauteil zu verstehen, das die HF-Elektroden trägt, mit denen HF-Energie in das zu behandelnde Körpergewebe eingekoppelt (übertragen) wird. Der Kopf trägt die HF-Elektroden. Sogenannte Herzkatheter sind spezielle Vorrichtungen für die Hochfrequenzbehandlung von Körpergewebe. Die vorliegende Erfindung betrifft insbesondere auch derartige Herzkatheter.

Die Entwicklung des Standes der Technik ging in den vergangenen Jahren insbesondere dahin, das Volumen des mittels HF-Energie denaturierten Gewebes zu erhöhen. Dies betrifft insbesondere die Entwicklung der genannten Herzkatheter. Hierzu wurden die aktiven Elektroden des Kopfes vergrößert. Mit zunehmendem Elektrodendurchmesser sowie größer werdenden Elektrodenflächen konnte das Volumen des denaturierten bzw. koagulierten Gewebes erhöht werden. Allerdings ist eine Erhöhung der von den HF-Elektroden abgegebenen HF-Energie nur soweit möglich, wie die Grenzschicht zwischen der HF-Elektrode und dem behandelten Gewebe bestimmte Temperaturen nicht übersteigt. Die im Bereich des Kontaktes zwischen der Elektrode und dem Gewebe erzeugte Temperatur ist wiederum davon abhängig, wie die entstehende Wärme aus diesem Bereich abgeführt wird. Die Elektrode selbst spielt bei dieser Wärmeabführung eine wesentliche Rolle. Auch das durchblutete Gewebe und das die Elektrode umströmende Blut führen Wärme ab. Durch Vergrößerung der aktiven Elektrode des HF-Kopfes läßt sich die Kontaktfläche mit dem Gewebe und damit auch die Kühlungsfläche vergrößern. Eine Vergrößerung der Elektrodenfläche ist aber für eine Vielzahl von Anwendungen nicht erwünscht.

Eine andere Möglichkeit der Kühlung wäre der Einsatz sogenannter aktiver Kühlsysteme, wie beispielsweise eine Durchspülung der Elektrode mit einer Kühlflüssigkeit, wie einer Kochsalzlösung. Die Kühlflüssigkeit könnte durch kleine Öffnungen in der Elektrode in die Blutbahn des Patienten eingeleitet werden. Dadurch wird aber der Blutkreislauf des Patienten belastet und das Infektionsrisiko erhöht. Auch wird hierdurch die Handhabung des HF-Gerätes wesentlich komplizierter.

Bei Vergrößerung der Elektrodenflächen werden durch die Abgabe der HF-Energie das Blut bzw. Gewebeteile weithin ungezielt erhitzt, d.h. es werden Blut und Gewebeteile stark erhitzt, bei denen dies nicht erwünscht ist. Das die Elektrodenflächen umspülende Blut kann zu kochen beginnen. Es wurde über bildgebende Verfahren (z.B. Ultraschall) eine extreme Blasenbildung an solchen Elektrodenteilen beobachtet, die nicht in direktem Kontakt mit zu behandelnden Gewebe waren. Eine derartige Blasenbildung im Blut oder auch an Gewebeoberflächen kann bei der Herzablation zu starken Artefakten im EKG-System führen. Auch eine Zerstörung von Blutkörperchen und eine Koagelbildung sind zu befürchten. Bei Hirnoperationen und Herzoperationen (Ablationstechnik) führen die beschriebenen Phänomene zu einer starken Beeinträchtigung der Ableitung von Signalen.

Der Erfindung liegt die Aufgabe zugrunde, eine Vorrichtung für die Hochfrequenzbehandlung von Körpergewebe der eingangs genannten Art so weiterzubilden, daß eine Zerstörung von Blutkörperchen und eine unerwünschte Koagelbildung sowie Verklumpung von Blut vermieden werden. Weiterhin soll der Wirkungsgrad der Vorrichtung, d.h. das Verhältnis von abgegebener HF-Energie zum erwünschten denaturierten Gewebevolumen verbessert werden.

Zur Lösung dieser technischen Probleme stellt die vorliegende Erfindung eine Vorrichtung gemäß Anspruch 1 bereit.

Die wenigstens eine HF-Elekrode ist auf dem Kopf ausgeformt. Der die HF-Elektrode abstützende Kopf weist hochwärmeleitfähige Bereiche auf, um an der HF-Elektrode entstehende Wärme von der Elektrode weg in proximaler Richtung abzuleiten, wobei der Kopf eine Länge von 6 bis 12 mm und einen Durchmesser von 1,5 bis 4 mm hat.

Hierdurch wird die Entstehung von Temperaturspitzen in der Nähe der Elektrode vermieden und die Wärme von den kritischen Stellen weggeleitet. Die Ableitung der Wärme kann auch teilweise in radialer Richtung (in bezug auf die Längsachse des Kopfes) erfolgen.

Die Erfindung ermöglicht die Verwendung von relativ kleinen aktiven Elektroden. Dadurch wird eine präzise Ableitung kleiner elektrischer Nervensignale und eine präzisere Lokalisierung von Nervenbahnen bzw. Gefäßen ermöglicht. Auch wird eine hohe Präzision hinsichtlich des Ortes der Gewebeimpedanzänderung während der HF-Abgabe erreicht. Weiterhin ermöglicht die Erfindung eine Verbesserung der Ableitungssignalqualität vor und während der HF-Abgabe. Auch die Temperaturmessung wird wesentlich verbessert und die Geschwindigkeit der Temperaturmessung in der aktiven Elektrode erhöht. Schließlich wird mit der Erfindung auch eine Anpassung der Elektrodenflächen an die zu behandelnden Gewebebereiche ermöglicht.

Nach der Erfindung wird bevorzugt die aktive Elektrode des Kopfes des HF-Gerätes nicht mehr wie beim Stand der Technik durch einen einzigen, massiven Metallkörper gebildet, der homogen in alle Richtungen HF-Energie abgibt, sondern durch eine oder mehrere kleinere leitfähige Elektrodenflächen, die sich auf das jeweils erwünschte Abstrahlungsgebiet beschränken. Dadurch ist eine präzise steuerbare HF-Energieabgabe in das tatsächlich zu behandelnde Gewebegebiet möglich. Blut und Gewebebereiche, die nicht mit HF-Energie belastet werden sollen, werden weniger stark erhitzt. Die Kühlung wird dadurch verbessert, daß die Gesamtfläche des Kopfes durch elektrisch isolierendes, aber thermisch hochleitfähiges Material vergrößert wird. Hierdurch wird auch die aktive Elektrodenfläche gekühlt.
Der erfindungsgemäße Kopf mit der HF-Elektrode oder mehreren HF-Elektroden ist derart hergestellt, daß der Kopf aus einem massiven Grundkörper aus thermisch hochleitfähigem Material, wie Diamant oder Keramik, gebildet wird, auf den die aktiven Elektrodenflächen als Schichten aufgebracht werden, z.B. durch Aufdampfung. Die aktiven Elektrodenflächen können direkt auch als Temperatursensoren ausgebildet werden (z.B. als Thermoelemente).

Alternativ kann der erfindungsgemäße Kopf auch so aufgebaut sein, daß er aus einem massiven Metallkörper besteht, der hochwärmeleitfähig ist und auf den eine elektrisch isolierende, aber thermisch hochleitfähige Schicht aufgebracht ist. Zum Beispiel kann diese Schicht aus Siliciumoxid, Diamant oder Keramik bestehen. Auf die elektrisch isolierende, thermisch hochleitfähige Schicht können dann die aktiven Elektroden ebenfalls als Schichten aufgebracht werden.

Der erfindungsgemäß vorgesehene ballige Elektrodenkopf hat insgesamt eine glatte Oberfläche und bei Ausbildung von zwei oder mehreren Elektrodenflächen sind diese unmittelbar benachbart, gegebenenfalls mit schmalen isolierenden Trennstreifen. Der Kopf weist außer den Elektrodenflächen noch weitere Flächen auf, die aus einem hochwärmeleitfähigen Material gebildet sind, um die Wärme wirksam aus dem Bereich der wirksamen Elektrodenflächen in weiter proximal gelegene Bereiche des Systems abzuleiten, wo die Wärme über größere Flächen und Volumina verteilt wird und so Temperaturspitzen vermieden sind. Gemäß bevorzugten Ausgestaltungen der Erfindung können die Elektroden als sehr dünne Schichten auf einen massiven Körper aufgebracht werden. Die Schichtdicke kann im Bereich weniger mm liegen, z. B. £ 5 mm.

Gemäß einer bevorzugten Ausgestaltung der Erfindung besteht der Kopf aus zumindest einer Elektrode und bis auf gegebenenfalls eine relativ dünne elektrisch isolierende Schicht ausschließlich aus hochwärmeleitfähigem Material.
Weitere bevorzugte Ausgestaltungen der Erfindung sind in den abhängigen Ansprüchen beschrieben.

So ist insbesondere vorgesehen, daß mehrere HF-Elektroden am distalen Ende des Kopfes angeordnet sind, wobei die einzelnen Elektroden wahlweise einzeln oder kombiniert angesteuert werden können, d.h. es kann je nach Bedarf die eine oder andere Elektrode ausgeschaltet werden, so daß sie keine HF-Energie abgibt. Mit einer solchen Gestaltung des Kopfes ist es möglich, nur diejenigen HF-Elektroden zu aktivieren, die tatsächlich so in Bezug auf das zu behandelnde Gewebe positioniert sind, daß die von ihnen abgegebene Energie die erwünschte Wirkung hat, während diejenigen HF-Elektroden, die nur eine unerwünschte Aufheizung von Blut oder Gewebe bewirken würden, nicht aktiviert werden.

Die Erfindung lehrt auch besondere Dimensionen für den Kopf und die Elektroden gemäß bevorzugten Ausführungsbeispielen.

Nachfolgend werden Ausführungsbeispiele der Erfindung anhand der Zeichnung näher beschrieben. Es zeigt:
- Fig. 1: ein erstes Ausführungsbeispiel einer Vorrichtung für die Hochfrequenzbehandlung von Körpergewebe im Axialschnitt und in einer Stirnansicht;
- Fig. 2: das Ausführungsbeispiel von Fig. 1 in Seitenansicht und in Stirnansicht;
- Fig. 3: die Wärmeleitung bei einer Vorrichtung gemäß den Fig. 1 und 2;
- Fig. 4: ein weiteres Ausführungsbeispiel einer Vorrichtung für Hochfrequenzbehandlung von Körpergewebe;
- Fig. 5: das Ausführungsbeispiel gemäß Fig. 4 im Schnitt;
- Fig. 6: eine Abwandlung des Ausführungsbeispiels gemäß den Fig. 4 und 5;
- Fig. 7: die Anwendung einer Vorrichtung gemäß den Fig. 4 bis 6 zur HF-Behandlung von Gewebe;
- Fig. 8: die Wärmeströmung bei Anwendung einer Vorrichtung gemäß Fig. 7;
- Fig. 9: ein weiteres Ausführungsbeispiel einer Vorrichtung für die Hochfrequenzbehandlung von Körpergewebe in Seitenansicht;
- Fig. 10: ein weiteres Ausführungsbeispiel einer Vorrichtung für die Hochfrequenzbehandlung von Körpergewebe im Schnitt; und
- Fig. 11: noch ein Ausführungsbeispiel einer Vorrichtung für die Hochfrequenzbehandlung von Körpergewebe.

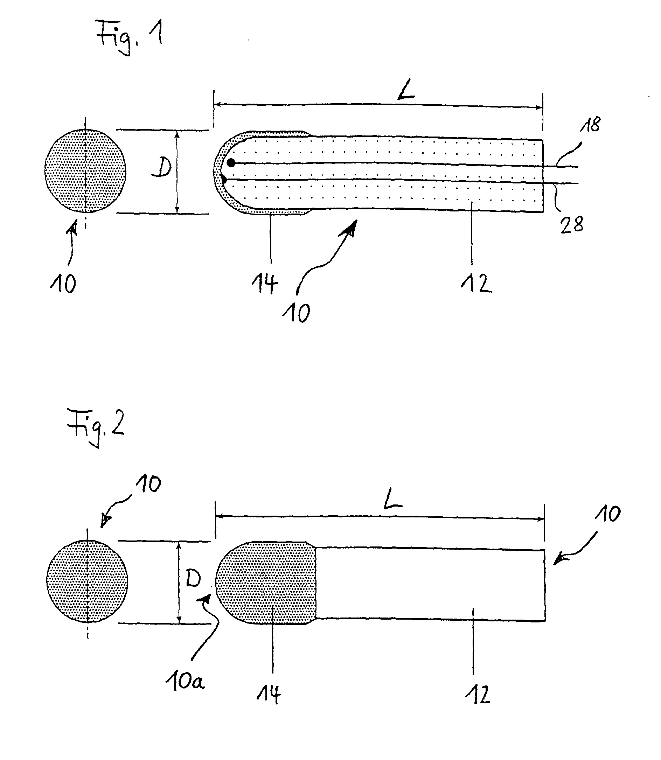

Fig. 1 zeigt einen Kopf 10 für ein HF-Behandlungsgerät. Der Kopf hat beim dargestellten Ausführungsbeispiel eine Länge L von ca. 8 mm und einen Durchmesser D von ca. 2 mm. Am distalen Ende des Kopfes 10 ist eine HF-Elektrode 14 in Form einer Metallschicht auf den Körper des Kopfes aufgebracht. Bis auf die metallische HF-Elektrode besteht also der Körper des Kopfes 10 aus Bereichen 12 aus hochwärmeleitfähigem Material, wie z.B. Diamant oder Keramik mit hoher Wärmeleitfähigkeit. Die Bereiche 12 sind elektrisch nicht leitfähig. Fig. 1 zeigt links auch eine Stirnansicht des Kopfes 10. Ein als solches bekannter Temperatursensor, z.B. ein Thermoelement, ist direkt proximal hinter der HF-Elektrode 14 in den Körper des Kopfes eingelassen und wird über eine Leitung 18 an die Steuer- und Versorgungseinrichtung (nicht gezeigt) angeschlossen. Die elektrische Leitung 28 für die HF-Elektrode ist ebenfalls in Fig. 1 schematisch dargestellt.
Fig. 2 zeigt die gleiche Vorrichtung wie Fig. 1, jedoch in nicht geschnittener Seitenansicht.

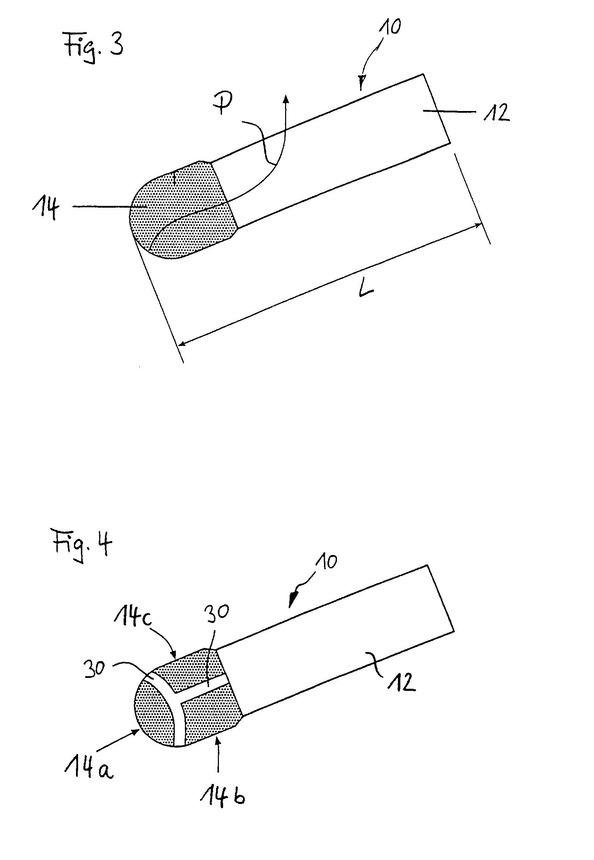

Fig. 3 zeigt die Wärmeleitung bei einer HF-Vorrichtung gemäß den Fig. 1 und 2. Wie eingangs ausgeführt ist, entsteht insbesondere auf der Oberfläche der HF-Elektrode Wärme, die es gilt, so zu verteilen, daß unnötige Temperaturspitzen vermieden werden. Die zuvor anhand der Fig. 1 und 2 beschriebene Struktur des Kopfes führt dazu, daß im Bereich der HF-Elektrode 14 entstehende Wärme nach hinten, d.h. in proximaler Richtung über die Bereiche 12 des Kopfes 10 entsprechend dem Pfeil P abgeleitet wird. Ein Temperaturstau an der distalen Spitze des Kopfes 10 wird dadurch vermieden.

Es wird also vermieden, daß insbesondere an der sogenannten Kontaktstelle, also in den Bereichen, in denen die Elektrode mit dem Gewebe in Berührung steht, eine unerwünschte Temperaturerhöhung stattfindet. Derartige Temperaturspitzen können im Extremfall sogar zu Gasbildung (verdampfendes Wasser) führen. Bei anhaltend unerwünschter Temperaturerhöhung trocknet die Kontaktfläche im Gewebebereich aus, wodurch der elektrische Kontakt (die Gewebeimpedanz) sehr schlecht werden und der Widerstand von z. B. 50 Ohm auf mehr als 1 Kiloohm ansteigen kann.

Durch die beschriebene Ableitung der Wärme aus der Metallspitze heraus in den wärmeleitenden Körper 12 des Kopfes 10 bleibt die Oberfläche der Elektrode 14 relativ kühl. Es entsteht auch im hinteren Teil (proximalen Teil) des Kopfes 10 keine unnötige Aufheizung von Körperflüssigkeit oder Körpergewebe durch HF-Energieabgabe, statt dessen ist der proximal hintere Teil des Kopfes zur Wärmeableitung und Kühlung vorgesehen. Der Wirkungsgrad des Kopfes, d.h. das Verhältnis von erwünschtem Koagulationsvolumen und HF-Energie ist günstig.

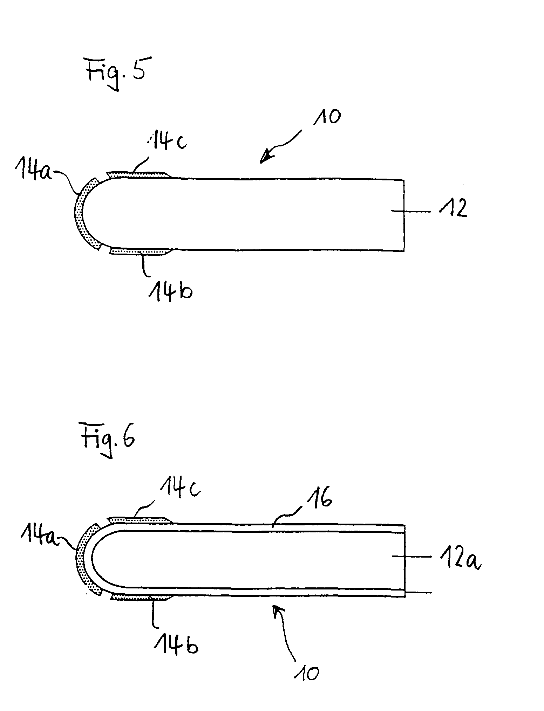

Figur 4 zeigt eine Weiterbildung des Ausführungsbeispiels gemäß den Figuren 1 bis 3. Bei der Vorrichtung für die HF-Behandlung gemäß Figur 4 sind am balligen distalen Ende des Kopfes 10 drei HF-Elektroden 14a, 14b, 14c ausgeformt. Eine Elektrode 14a ist an der balligen Spitze in Form einer Kappe als Metallschicht aufgebracht und zwei weitere Elektroden 14b, 14c sind unmittelbar proximal dahinter gemäß Fig. 4 ausgeformt. Dreht man den Kopf 10 gemäß Fig. 4 um seine Längsachse um 180° entsteht das gleiche Bild wie in Fig. 4 gezeigt ist. Zwischen den Elektroden 14a, 14b und 14c sind schmale Streifen 30 aus elektrisch isolierendem Material ausgebildet. Das Material der Streifen 30 ist ebenso wie das Material der restlichen Bereiche 12 des Kopfes 10 aus hochwärmeleitfähigem Material.

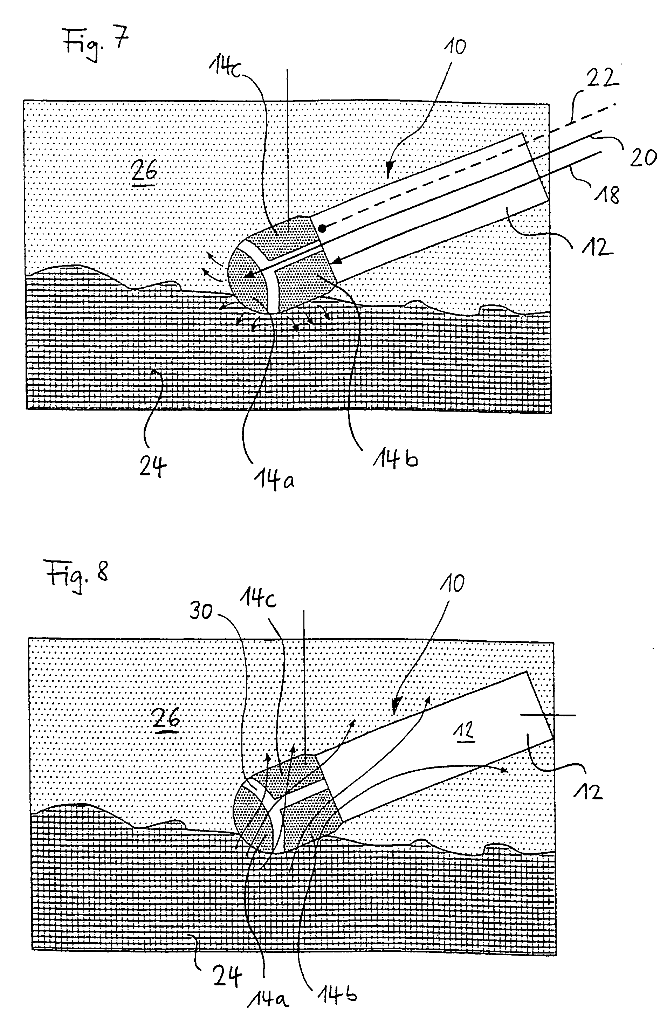

Die mehreren Elektroden, beim Ausführungsbeispiel nach Fig. 4 drei Elektroden 14a, 14b und 14c, sind wahlweise einzeln aktivierbar, d.h. es können wahlweise eine, zwei oder auch alle drei Elektroden zur Abgabe von HF-Energie mit Strom versorgt werden, je nach den Einsatzbedingungen des Gerätes. Dies wird anhand der Fig. 7 und 8 weiter unten näher erläutert.

Die einzelnen Elektroden 14a, 14b und 14c sind jeweils mit einem eigenen Temperatursensor ausgerüstet, so daß die unmittelbar an den Elektroden bzw. Elektrodenflächen entstehenden Temperaturen weitgehend unverfälscht gemessen werden können. Weiterhin kann zumindest ein Temperatursensor so angeordnet werden, daß er die Temperatur der thermisch hochwärmeleitfähigen Bereiche 12 des Kopfes 10 mißt. Diese einzelnen Temperatursensoren (z.B. Thermoelemente) sind in den Figuren nicht besonders dargestellt. Es ist auch möglich, die Temperatursensoren durch sogenannte Thermistoren zu verwirklichen. Alternativ ist es auch möglich, als Temperatursensoren Infrarotdetektoren, fasertechnische Aufnehmer oder auch temperaturabhängig schwingende Elemente vorzusehen, die ihre Farbreflexion oder Farbstrahlung in Abhängigkeit von der Temperatur ändern.

Bei Verwendung eines Thermoelementes werden zwei verschiedene Metalle, die sich berühren, verwendet, z. B. Gold und NiCr. Dies kann z. B. derart verwirklicht werden, daß die Keramikoberfläche mit Palladium als Haftträger bedampft wird und anschließend eine NiCr-Schicht aufgetragen wird. Letztere wird mit Gold überzogen. Die Fläche zwischen der NiCr-Schicht und der Goldschicht bildet den Temperatursensor in Form eines Flächensensors.

Bei Verwendung eines Infrarotsensors läßt sich die Temperatur direkt messen, nämlich als Oberflächentemperatur. Das Infrarotsignal kann über geeignete IR-leitende Glas- oder Quarzfasern auch aus extrem dünnen Elektroden gewonnen und zu einem voluminöseren Infrarotdetektor weitergeleitet werden.

Die genannten fasertechnischen Aufnehmer verwenden eine Glasfaser, die im Frontbereich (sogenanntes erstes Ende) mit temperatursensiblen fluoreszierenden oder reflektierenden Stoffen beschichtet ist. Diese Stoffe verändern in Abhängigkeit von der Temperatur ihre farbreflektierenden oder fluoreszierenden Eigenschaften. Durch Einkopplung von Licht über das zweite Ende der Faser lassen sich die Stoffe anregen, z.B. ihre reflektierende Eigenschaft ausnutzen.

Es ist auch möglich, die Temperaturmessung über eine Längenänderung einer kurzen Faser durchzuführen. Dabei wird ein kleiner Faserkopf (1 bis 2 mm lang) an beiden Enden verspiegelt. Ein Ende verfügt dabei über einen halbdurchlässigen Spiegel. Über einen eingekoppelten, abstimmbaren Laserstrahl läßt sich nun die Resonanz des verspiegelten Faserkopfes messen. Durch Temperaturänderung des Faserkopfes verändert sich dessen Länge und damit auch dessen Resonanz.

Temperaturabhängig schwingende Elemente können z. B. durch Quarzmaterialien verwirklicht werden. Quarze werden hierfür so gezüchtet, daß ihr thermisches Verhalten einen möglichst großen Einfluß auf die Eigenresonanzfrequenz hat.

Als Material für die hochwärmeleitfähigen Bereiche 12 des Kopfes 10 kommen insbesondere keramische Materialien, Glaskeramik, Siliziumoxid und Diamant in Betracht. Auf diese Materialien als Grundmaterial des Körpers des Kopfes 10 werden dann die HF-Elektroden flächig aufgebracht oder in geeigneter Weise in dieses Material eingebettet.

Fig. 5 zeigt eine solche Struktur im Schnitt, wobei der Körper 12 des Kopfes 10 aus hochwärmeleitfähigem, aber elektrisch isolierendem Material besteht und die Elektroden 14a, 14b, 14c aus elektrisch leitfähigem Material in Schichtform aufgebracht sind, z. B. durch Aufdampfung.

Fig. 6 zeigt eine Abwandlung der zuvor beschriebenen Ausführungsbeispiele mit einem Hauptkörper 12a des Kopfes 10 aus hochwärmeleitfähigem Metall, wie z. B. Kupfer. Auf den wärmeleitfähigen Hauptkörper 12a ist eine dünne elektrisch isolierende Schicht 16 aus ebenfalls gut wärmeleitendem Material aufgebracht, wie z. B. Diamant oder Keramik. Auf die elektrisch isolierende, aber gut wärmeleitende Schicht 16 sind die Elektroden 14a, 14b, 14c als metallisierte Flächen aufgebracht, z. B. aus Platin oder Gold.

Die Fig. 7 und 8 erläutern den Betrieb eines HF-Gerätes gemäß den Fig. 4 bis 6. Es soll Gewebe 24 mit HF-Energie behandelt werden, z. B. eine Herzwand. Das Blut ist mit dem Bezugszeichen 26 angedeutet. Wie Fig. 7 zeigt, sind nur die Elektroden 14a und 14b in direktem Kontakt mit dem zu behandelnden Gewebe 24. Deshalb werden nur die HF-Leitungen 18 und 20 aktiviert, die die Elektroden 14b bzw. 14a mit Strom versorgen. Die Erzeugung und der Transport der HF-Energie ist in Fig. 7 mit den kleinen Pfeilen angedeutet, die sich von den aktivierten Elektroden 14a, 14b wegerstrecken. Die nicht aktivierte Elektrode 14c, deren Zuleitung 22 ohne Strom bleibt, erhitzt das umgebende Blut 26 nicht unnötig.

Fig. 8 zeigt den Wärmetransport schematisch mit den dargestellten Teilen. Wie zu erkennen ist, wirken nicht nur die proximalen Bereiche 12 des Kopfes 10 wärmeabführend, sondern auch die nicht aktivierte Elektrode 14c und auch die elektrisch isolierenden Streifen 30 zwischen den einzelnen Elektroden. Bei einer HF-Behandlungsvorrichtung gemäß Fig. 6 ist die Wirkung ähnlich der Fig. 8.

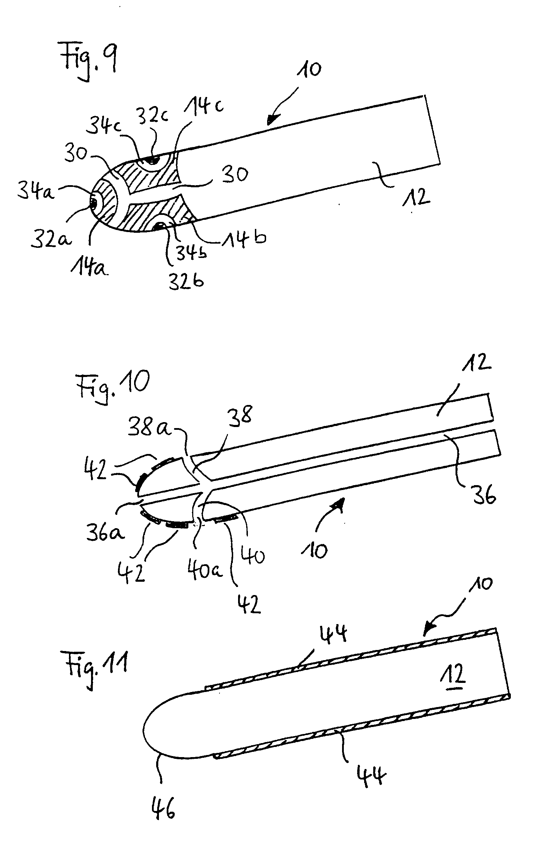

Die Fig. 9, 10 und 11 zeigen weitere Ausführungsbeispiele von Vorrichtungen für die HF-Behandlung von Körpergewebe.

Das Ausführungsbeispiel gemäß Fig. 9 bildet die vorstehend beschriebenen Ausführungsbeispiele dahingehend weiter, daß der Kopf durch ein inneres Kühlsystem weiter gekühlt wird. Für die Kühlung des Kopfes sind im Kopf 10 Kanäle ausgeformt, durch die eine Kühlflüssigkeit strömt. Die Kühlflüssigkeit kann durch den Kopf strömen, ohne diesen zu verlassen. Es ist aber auch möglich, gemäß dem Ausführungsbeispiel nach Fig. 9 die Kühlflüssigkeit durch Öffnungen 32a, 32b, 32c aus dem Kopf austreten zu lassen. Die Öffnungen 32a, 32b und 32c sind jeweils etwa mittig in einer Elektrode 14a, 14b bzw. 14c angeordnet. Die Löcher sind jeweils von Bereichen 34a, 34b und 34c aus elektrisch isolierendem Material umgeben. Das genannte elektrisch isolierende Material ist aber hochwärmeleitfähig.

Fig. 10 zeigt eine ähnliche Kühlanordnung für einen HF-Kopf 10 im Schnitt. Die Kanäle für Kühlflüssigkeit sind mit den Bezugszeichen 36, 38 und 40 versehen. Die Öffnungen der Kanäle mit den Bezugszeichen 36a, 38a bzw. 40a. Fig. 10 zeigt auch aufgedampfte Elektroden 42. Bei diesem Ausführungsbeispiel, wie auch bei den zuvor beschriebenen Ausführungsbeispielen, kann es sich bei den Elektroden um sehr dünn aufgedampfte Schichten handeln. In den Fig. sind die Schichtdicken der Elektroden zur Verdeutlichung übertrieben stark dargestellt. Z. B. kann die Schichtdicke der Elektroden 42 nur wenige mm, insbesondere weniger als 10 mm, besonders bevorzugt weniger als 4 mm betragen.

Es ist mit moderner Festkörpertechnik möglich, in einem einzigen Körper Bereiche mit unterschiedlicher elektrischer Leitfähigkeit auszubilden. So können z. B. in Diamant durch Dotierung mit unterschiedlichen Atomen während des Wachstums elektrisch leitende Bereiche und elektrisch nicht leitende Bereiche erzeugt werden. Auf diese Weise können sogar Elektrodenbereiche und Zuleitungen zu den Elektroden integral in einem wachsenden Diamant-Kristall ausgebildet werden.

Fig. 11 zeigt ein weiteres Ausführungsbeispiel, bei dem der Kopf 10 insgesamt aus einem Metall besteht, auf welches eine elektrisch isolierende, aber thermisch sehr gut leitende Schicht 44 aufgebracht ist. Die distale Spitze 46 des Metallkörpers dient als leitende HF-Elektrode.

Besondere Aspekte der vorstehend beschriebenen Erfindung lassen sich also wie folgt zusammenfassen:

Bei der HF-Vorrichtung ist zumindest eine HF-Elektrode am distalen Ende des Kopfes angeordnet.

Weiterhin kann vorgesehen sein, daß der Kopf zylindrisch mit balliger distaler Spitze 10a geformt ist.

Weiterhin sind zwei oder mehr Elektroden (14a, 14b, 14c) am Kopf (10) vorgesehen, die wahlweise aktivierbar sind. Auch kann vorgesehen sein, daß die HF-Elektrode (14) bzw. die HF-Elektroden (14a, 14b, 14c) auf einen Körper aus leitfähigem Material aufgebracht sind, gegebenenfalls mit einer elektrisch isolierenden Schicht (16) dazwischen.

Dabei kann vorgesehen sein, daß die HF-Elektrode bzw. die HF-Elektroden in einem Bereich von 1 bis 6 mm, bevorzugt 2 bis 4 mm, der Längserstreckung des Kopfes im Bereich von dessen distalem Ende angeordnet sind.

Ein anderer Aspekt der Erfindung liegt insbesondere darin, daß drei oder mehr HF-Elektroden (14a, 14b, 14c) am Kopf (10) angeordnet sind, eine davon am distalen Ende (10a) und die weiteren Elektroden proximal dahinter.

Eine andere Ausgestaltung sieht vor, daß ein Temperatursensor oder mehrere Temperatursensoren vorgesehen sind, um die Temperatur des thermisch hochleitfähigen Bereichs (12) des Kopfes (10)zu messen.

Dabei kann der Temperatursensor bzw. können die Temperatursensoren durch Thermoelemente gebildet sein.

Als Temperatursensor können auch Thermistoren vorgesehen sein.

Weiterhin kann vorgesehen sein, daß zumindest einer der Temperatursensoren durch einen Infrarotdetektor oder einen fasertechnischen Aufnehmer oder temperaturabhängig schwingende Elemente gebildet ist, wobei letztere ihre Farbreflexion oder Farbstrahlung in Abhängigkeit von der Temperatur ändern.

Ein anderer Aspekt der Erfindung liegt darin, daß der Körper 12 des Kopfes ebenso wie die HF-Elektroden aus Metall gebildet sind, wobei die HF-Elektroden durch elektrisch isolierende Streifen (30) bzw. Schichten (16) vom Körper (12a) getrennt sind und letzterer aus thermisch hochleitfähigem Metall gebildet ist, z. B. Kupfer.

Weiterhin kann vorgesehen sein, daß die wirksamen Oberflächen der elektrisch leitfähigen HFElektroden durch Aufbringen von elektrisch isolierendem Material reduziert sind.

Weiterhin kann im Inneren des Kopfes ein Kühlsystem vorgesehen sein mit einem strömenden Kühlmedium.

Dabei kann die Kühlflüssigkeit durch eine oder mehrere Öffnungen aus dem Kopf nach außen dringen.

Weiterhin kann vorgesehen sein, daß mehrere zusätzliche Elektroden in proximaler Richtung angeordnet sind, welche wiederum in Teilsegmente unterteilt sein können.

## Patentansprüche

1. Vorrichtung für die Hochfrequenzbehandlung von Körpergewebe mit einem Kopf (10), der zumindest eine HF-Elektrode (14) aus elektrisch leitfähigem Material aufweist, die mit dem zu behandelnden Gewebe (24) in Kontakt bringbar ist,
wobei der die HF-Elektrode (14) abstützende Kopf (10) aus wärmeleitfähigem Material besteht, um an der HF-Elektrode (14) entstehende Wärme in proximaler Richtung abzuleiten, wobei der Kopf (10) eine Länge von 6 bis 12 mm hat,
**dadurch gekennzeichnet, dass** der kopf (10) einen Durchmesser von 1,5 bis 4 mm hat und
der Kopf (10) aus einem massiven Grundkörper gebildet wird, auf dem die mindestens eine HF-Elektrode (14) als Schicht aufgebracht ist.

2. Vorrichtung nach Anspruch 1,
**dadurch gekennzeichnet,**
**daß** die hochwärmeleitfähigen Bereiche (12) aus elektrisch isolierendem Material bestehen.

3. Vorrichtung nach Anspruch 1 oder 2,
**dadurch gekennzeichnet,**
**daß** der Kopf eine Länge von 7 bis 10 mm hat.

4. Vorrichtung nach einem der Ansprüche 1 bis 3,
**dadurch gekennzeichnet,**
**daß** der Kopf einen Durchmesser von 1,5 bis 3 mm hat.

5. Vorrichtung nach einem der vorhergehenden Ansprüche
**dadurch gekennzeichnet,**
**daß** zumindest eine der Elektroden (14) im Bereich der Spitze (10a) des Kopfes (10) angeordnet ist.

6. Vorrichtung nach einem der vorhergehenden Ansprüche,
**dadurch gekennzeichnet,**
**daß** die Bereiche (12) des Kopfes aus hochwärmeleitfähigem Material eine wärmeableitende Oberfläche haben, die 0,3 bis 10 mal so groß ist wie die wirksame Oberfläche der HF-Elektrode (14) oder der HF-Elektroden (14a, 14b, 14c).

7. Vorrichtung nach Anspruch 6,
**dadurch gekennzeichnet,**
**daß** die Bereiche des Kopfes aus hochwärmeleitfähigem Material eine wärmeableitende Oberfläche haben, die 1 bis 2 mal so groß ist wie die wirksame Oberfläche der HF-Elektrode (14) oder der HF-Elektroden (14a, 14b, 14c).

8. Vorrichtung nach einem der vorhergehenden Ansprüche,
**dadurch gekennzeichnet,**
**daß** die hochwärmeleitfähigen Bereiche (12) des Kopfes (10) aus Diamant oder Keramik bestehen.

9. Vorrichtung nach einem der vorhergehenden Ansprüche, **dadurch gekennzeichnet,**
**daß** die eine HF-Elektrode (14) oder die mehreren HF-Elektroden (14a, 14b, 14c) jeweils mit einem Temperatursensor versehen sind.

10. Vorrichtung nach einem der vorhergehenden Ansprüche,
**dadurch gekennzeichnet,**
**daß** eine zusätzliche HF-Elektrode in proximaler Richtung vorgesehen ist, wobei die zusätzliche Elektrode in einzeln ansteuerbare Segmente unterteilt sein kann.

## Claims

1. An apparatus for the high-frequency treatment of body tissue with a head (10) which comprises at least one HF electrode (14) of an electrically conductive material that can be brought into contact with the tissue (24) to be treated, wherein the head (10) supporting the HF electrode (14) consists of heat-conductive material in order to dissipate the heat generating at the HF electrode (14) in the proximal direction, the head (10) having a length of 6 to 12 mm, **characterized in that** the head (10) has a diameter of 1.5 to 4 mm and is comprised of a massive basic body on which the at least one HF electrode (14) is applied as a layer.

2. The apparatus according to Claim 1,
**characterised in that** the highly heat-conductive portions (12) consist of an electrically insulating material.

3. The apparatus according to Claim 1 or 2,
**characterised in that** the head has a length of 7 to 10 mm.

4. The apparatus according to one of Claims 1 to 3,
**characterised in that** the head has a diameter of 1.5 to 3 mm.

5. The apparatus according to one of the previous claims,
**characterised in that** at least one of the electrodes (14) is arranged in the area of the tip (10a) of the head (10).

6. The apparatus according to one of the previous claims,
**characterised in that** the portions (12) of the head of a highly heat-conductive material have a heat dissipating surface which is 0.3 to 10 times the effective surface of the HF electrode (14) or the HF electrodes (14a, 14b, 14c).

7. The apparatus according to Claim 6,
**characterised in that** the portions of the head of a highly heat-conductive material have a heat dissipating surface which is 1 to 2 times the effective surface of the HF electrode (14) or the HF electrodes (14a, 14b, 14c).

8. The apparatus according to one of the previous claims,
**characterised in that** the highly heat-conductive portions (12) of the head (10) consist of diamond or ceramic.

9. The apparatus according to one of the previous claims,
**characterised in that** the one HF electrode (14) or the several HF electrodes (14a, 14b, 14c) is/are provided with a temperature sensor each.

10. The apparatus according to one of the previous claims,
**characterised in that** an additional HF electrode is provided in the proximal direction, which additional electrode may be divided into individually controllable segments.

## Revendications

1. Dispositif pour le traitement haute fréquence de tissus corporels, pourvu d'une tête (10) présentant pour le moins une électrode HF (14) en matériau électroconductible, laquelle électrode peut être mise en contact avec le tissu à traiter (24),
la tête (10) supportant l'électrode HF (14) étant composée d'un matériau électroconductible de manière à dissiper en direction proximale la chaleur générée à l'électrode HF (14), la tête (10) ayant une longueur de 6 à 12 mm,
**caractérisé en ce que** la tête (10) a un diamètre de 1,5 à 4 mm et est formée par un corps de base massif sur lequel ladite électrode HF (14) est appliquée en couche.

2. Dispositif selon la revendication 1,
**caractérisé en ce que**
les zones à haute conductibilité thermique (12) sont constituées d'une matière électro-isolante.

3. Dispositif selon la revendication 1 ou 2,
**caractérisé en ce que**
la tête a une longueur de 7 à 10 mm.

4. Dispositif selon l'une des revendications 1 à 3,
**caractérisé en ce que**
la tête a un diamètre de 1,5 à 3 mm.

5. Dispositif selon l'une des revendications précédentes,
**caractérisé en ce que**
pour le moins une des électrodes (14) est disposée dans la zone de la pointe (10a) de la tête (10).

6. Dispositif selon l'une des revendications précédentes,
**caractérisé en ce que**
les zones (12) de la tête constituées d'un matériau à haute conductibilité thermique présentent une surface dissipatrice de chaleur qui est de 0,3 à 10 fois supérieure à la surface active de l'électrode HF (14) ou des électrodes HF (14a, 14b, 14c).

7. Dispositif selon la revendication 6,
**caractérisé en ce que**
les zones de la tête constituées d'un matériau à haute conductibilité thermique présentent une surface dissipatrice de chaleur qui est de 1 à 2 fois supérieure à la surface active de l'électrode HF (14) ou des électrodes HF (14a, 14b, 14c).

8. Dispositif selon l'une des revendications précédentes,
**caractérisé en ce que**
les zones à haute conductibilité thermique (12) de la tête (10) sont en diamant ou en céramique.

9. Dispositif selon l'une des revendications précédentes,
**caractérisé en ce que**
l'électrode HF (14) ou les électrodes HF (14a, 14b, 14c) sont équipées chacune d'un capteur de température.

10. Dispositif selon l'une des revendications précédentes,
**caractérisé en ce que**
une électrode HF supplémentaire est prévue en direction proximale, l'électrode supplémentaire pouvant être divisée en plusieurs segments commandables séparément.
